# EUROPEAN PATENT APPLICATION

(11) **EP 3 150 191 A1**
(43) Date of publication of application: **05.04.2017**
(21) Application number: 15798902.1
(22) Date of filing: 29.05.2015
(51) Int. Cl.: A61K 8/72, A61K 8/06, A61K 8/02, A61Q 19/00

(54) **COSMETIC COMPOSITION CONTAINING AMPHIPHILIC ANISOTROPIC POWDER AND METHOD FOR PREPARING SAME**

(30) Priority: 30.05.2014 KR 20140066004; 14.05.2015 KR 20150067651
(71) Applicant: Amorepacific Corporation, Seoul 140-777 (KR)
(72) Inventor: NAM, Jin, Yongin-si Gyeonggi-do 446-729 (KR); KIM, Youngsun, Yongin-si Gyeonggi-do 446-729 (KR); JIN, Yu Jin, Yongin-si Gyeonggi-do 446-729 (KR); PI, Bongsoo, Yongin-si Gyeonggi-do 446-729 (KR); AN, Soon Ae, Yongin-si Gyeonggi-do 446-729 (KR); KANG, Byung Young, Yongin-si Gyeonggi-do 446-729 (KR); HAN, Sang Hoon, Yongin-si Gyeonggi-do 446-729 (KR)
(74) Representative: Agasse, Stéphane
(86) International application number: PCT/KR2015/005432
(87) International publication number: WO 2015/183042

(57) **Abstract**

Disclosed are a cosmetic composition containing an amphiphilic anisotropic powder and a method for preparing the same. The cosmetic composition contains an amphiphilic anisotropic powder which maximizes chemical interface activity and physical interface activity by controlling amphiphilic property, which is the characteristic of an existing surfactant, and geometric property, which is the characteristic of powder macroparticles, thereby forming an emulsion having excellent and stabilized emulsifying capacity.

## Description

### [Technical Field]

The present disclosure relates to a cosmetic composition including amphiphilic anisotropic powder and a method for preparing the same.

### [Background Art]

A surfactant forms a curved surface toward an oil phase or an aqueous phase depending on its packing parameters, and one portion of surfactant is subjected to hydration with an aqueous phase and the other portion thereof is subjected to solvation with oil, thereby forming water-in-oil (W/O) type or oil-in-water (O/W) type emulsion.

Meanwhile, Pickering emulsion using spherical solid powder unlike the conventional molecular surfactant forms W/O or O/W emulsion depending on the wettability at the interface with the solid powder surface, i.e., degree of oelophilicity or hydrophilicity. One factor determining the orientability of a surface film is contact angle. When the contact angle is smaller than 90°, a larger portion of particle surfaces exists as an aqueous phase to form O/W emulsion. On the other hand, when the contact angle is larger than 90°, a larger portion of particle sizes exists in an oil phase to form W/O emulsion.

In general, as compared to the conventional surfactant system, a Pickering emulsion allows formation of macroemulsion particles and the formed emulsion particles are prevented from coalescence by virtue of physical stabilization, thereby providing stabilized emulsion particles. Therefore, many studies have been conducted about a method for varying physical properties of emulsion particles depending on the particle size and property of Pickering solid particles and about investigation of physical properties varied thereby. However, although Pickering solid particles have a hydrophilic or oleophilic surface, they have no amphiphilic property, unlike a surfactant.

Therefore, there have been many attempts to enhance surface active property of spherical powder particles used in Pickering by imparting amphiphilic surface active property to the particles. Particular examples of such an attempt include Janus particles. However, such attempts have not yet been applied practically due to geometrical limit and difficulty in mass production with uniform quality.

Korean Patent Laid-Open No.1997-0025588 is incorporated herein by reference.

### [Disclosure]

### [Technical Problem]

A technical problem to be solved by the present disclosure is to provide a cosmetic composition that forms a stabilized emulsion having high emulsification capability by using amphiphilic anisotropic powder having maximized chemical surface active property and physical surface active property through the introduction of amphiphilic interfacial property to anisotropic polymer powder.

### [Technical Solution]

In one aspect, there is provided a cosmetic composition including amphiphilic anisotropic powder, wherein the powder includes a first hydrophilic polymer spheroid and a second hydrophobic polymer spheroid, the first polymer spheroid and the second polymer spheroid are bound with each other in such a manner that one polymer spheroid at least partially penetrates the other polymer spheroid, and the first polymer spheroid has a core-shell structure and the shell includes a functional group.

According to an embodiment, the core of the first polymer spheroid and the second polymer spheroid may include a vinyl polymer and the shell of the first polymer spheroid may include a copolymer of a vinyl monomer with a functional group.

According to another embodiment, the vinyl polymer may be a vinyl aromatic polymer.

According to still another embodiment, the functional group may be a siloxane.

According to still another embodiment, the shell of the first polymer spheroid may further include a hydrophilic functional group introduced thereto.

According to still another embodiment, the hydrophilic functional group may be at least one selected from the group consisting of a carboxylic acid group, sulfone group, phosphate group, amino group, alkoxy group, ester group, acetate group, polyethylene glycol group and a hydroxyl group.

According to still another embodiment, the amphiphilic anisotropic powder may have a symmetric form, asymmetric snowman form or asymmetric reversed snowman form on the basis of the binding portion where the first polymer spheroid and the second polymer spheroid are bound with each other.

According to still another embodiment, the amphiphilic anisotropic powder may have a particle size of 100-2500 nm.

According to still another embodiment, the amphiphilic anisotropic powder may form macroemulsion particles with a size of 2-200 µm.

According to still another embodiment, the cosmetic composition may have a formulation of an oil-in-water (O/W) type or water-in-oil (W/O) type, or a multiple formulation of W/O/W type or O/W/O.

According to yet another embodiment, the amphiphilic anisotropic powder may be used in an amount of 0.1-15 wt% based on the total weight of cosmetic composition.

In another general aspect, there is provided a method for preparing the cosmetic composition, including the steps of: (1) agitating a first monomer with a polymerization initiator to form a core of the first polymer spheroid; (2) agitating the resultant core of the first polymer spheroid with a first monomer, polymerization initiator and a functional group-containing compound to form a coated first polymer spheroid having a core-shell structure; (3) agitating the resultant first polymer spheroid having a core-shell structure with a second monomer and polymerization initiator to provide anisotropic powder in which a second polymer spheroid is formed; (4) introducing a hydrophilic functional group to the resultant anisotropic powder to provide amphiphilic anisotropic powder; and (5) carrying out emulsification by using the resultant amphiphilic anisotropic powder.

According to an embodiment, in steps (1)-(3), the agitation may be carried out by rotary agitation in a cylindrical reactor.

According to an embodiment, in step (1), the first monomer may be mixed with the polymerization initiator at a weight ratio of 100-1000:1.

According to another embodiment, in step (2), the functional group-containing compound may be siloxane-containing (meth)acrylate.

According to still another embodiment, in step (2), the first monomer, polymerization initiator and the functional group-containing compound may be mixed at a weight ratio of 80-98:0.2-1.0:1-20.

According to still another embodiment, in step (3), the second monomer and polymerization initiator may be mixed at a weight ratio of 150-250:1.

According to still another embodiment, in step (4), the hydrophilic functional group may be introduced by using a silane coupling agent.

### [Advantageous Effects]

According to the embodiments of the present disclosure, it is possible to provide a cosmetic composition that forms a stabilized emulsion having high emulsification capability by using amphiphilic anisotropic powder having maximized chemical surface active property simultaneously with maximized physical surface active property through the control of the amphiphilic property of a known surfactant and the geometrical property of macropowder particles.

In addition, according to the embodiments of the present disclosure, it is possible to provide a cosmetic composition that can be provided in various formulations and has a matte feel of use with no stickiness or irritatability caused by the conventional surfactant.

### [Description of Drawings]

Fig. 1 is a schematic view showing the emulsification of amphiphilic anisotropic powder according to an embodiment.
Fig. 2 shows optical microscopic images of emulsion (O/W) compositions of anisotropic powder including various types of oil (25%) according to an embodiment (scale bar: 10 µm).
Fig. 3 shows optical microscopic images of emulsion compositions depending on content of anisotropic powder of the cosmetic composition according to an embodiment (scale bar: 10 µm).
Fig. 4 shows images illustrating variations in formulation (O/W, W/O) depending on oil/water composition in the anisotropic powder-emulsified composition of the cosmetic composition according to an embodiment (scale bar: 20 µm).
Fig. 5 shows photographic images illustrating an emulsification capability test of the cosmetic composition according to an embodiment (scale bar: 10 µm).
Fig. 6 shows photographic images illustrating emulsion particles in the cosmetic composition according to an embodiment as observed by the naked eye.
Fig. 7 shows photographic images illustrating oil-in-water emulsion particles of the cosmetic composition according to an embodiment and oil-in-water emulsion particles of the conventional surfactant system (scale bar: 10 µm).
Fig. 8 shows optical microscopic images of multiple formulations of the cosmetic composition according to an embodiment.

### [Best Mode]

Exemplary embodiments now will be described in detail hereinafter.

As used herein, unless otherwise defined, the term 'substituted' means that at least one hydrogen atom of the functional group disclosed herein is substituted with a halogen (F, Cl, Br or I), hydroxyl group, nitro group, imino group (=NH, =NR, wherein R is a C1-C10 alkyl group), amidino group, hydrazine or hydrazone group, carboxyl group, substituted or non-substituted C1-C20 alkyl group, substituted or non-substituted C3-C30 heteroaryl group, or a substituted or non-substituted C2-C30 heterocycloalkyl group.

As used herein, the term '(meth)acryl' means acryl and/or methacryl.

As used herein, the particle size of amphiphilic powder is determined by measuring the maximum length of powder particles.

In one aspect, there is provided a cosmetic composition including amphiphilic anisotropic powder, wherein the powder includes a first hydrophilic polymer spheroid and a second hydrophobic polymer spheroid, wherein the first polymer spheroid and the second polymer spheroid are bound with each other in such a manner that one polymer spheroid at least partially penetrates the other polymer spheroid, the first polymer spheroid has a core-shell structure and the shell includes a functional group.

As used herein, 'spheroid' means a body including a polymer. For example, a spheroid may be a sphere, globoid or oval shape and have a micro-scale or nano-scale long-axis length on the basis of the largest length in the section of body.

According to an embodiment, the core of the first polymer spheroid and the second polymer spheroid may include a vinyl polymer and the shell of the first polymer spheroid may include a copolymer of a vinyl monomer with a functional group.

According to another embodiment, the vinyl polymer may be a vinyl aromatic polymer, such as polystyrene.

According to still another embodiment, the functional group may be a siloxane.

According to still another embodiment, the shell of the first polymer spheroid may further include a hydrophilic functional group introduced thereto.

According to still another embodiment, the hydrophilic functional group may be a negatively charged or positively charged functional group or PEG (polyethylene glycol)-based functional group, and may be at least one selected from the group consisting of a carboxylic acid group, sulfone group, phosphate group, amino group, alkoxy group, ester group, acetate group, polyethylene glycol group and a hydroxyl group.

According to still another embodiment, the amphiphilic anisotropic powder may have a symmetric form, asymmetric snowman form or asymmetric reversed snowman form on the basis of the binding portion where the first polymer spheroid and the second polymer spheroid are bound with each other. The term 'snowman form' means one having the first polymer bound with the second polymer, wherein the first polymer and the second polymer have a different size.

According to still another embodiment, the amphiphilic anisotropic powder may have a particle size of 100-2500 nm. According to still another embodiment, the amphiphilic powder may have a particle size of 100-1500 nm, 100-500 nm, or 200-300 nm. Particularly, the amphiphilic powder may have a particle size of at least 100 nm, at least 200 nm, at least 300 nm, at least 400 nm, at least 500 nm, at least 600 nm, at least 700 nm, at least 800 nm, at least 900 nm, at least 1000 nm, at least 1100 nm, at least 1200 nm, at least 1300 nm, at least 1400 nm or at least 1500 nm, and at most 2500 nm, at most 2400 nm, at most 2300 nm, at most 2200 nm, at most 2100 nm, at most 2000 nm, at most 1900 nm, at most 1800 nm, at most 1700 nm, at most 1600 nm, at most 1500 nm, at most 1400 nm, at most 1300 nm, at most 1200 nm, at most 1100 nm, at most 1000 nm, at most 900 nm, at most 800 nm, at most 700 nm, at most 600 nm, at most 500 nm, at most 400 nm, at most 300 nm or at most 200 nm.

According to still another embodiment, the amphiphilic anisotropic powder may form macroemulsion particles with a size of 2-200 µm. According to yet another embodiment, the amphiphilic powder may form macroemulsion particles with a size of 5-200 µm, 10-100 µm, 10-50 µm, or 25 µm. Particularly, the amphiphilic powder may form emulsion particles with a size of at least 2 µm, at least 5 µm, at least 10 µm, at least 15 µm, at least 20 µm, at least 25 µm, at least 30 µm, at least 40 µm, at least 50 µm, at least 80 µm, at least 100 µm, at least 130 µm, at least 150 µm or at least 180 µm, and at most 200 µm, at most 180 µm, at most 150 µm, at most 130 µm, at most 100 µm, at most 80 µm, at most 50 µm, at most 40 µm, at most 30 µm, at most 25 µm, at most 20 µm, at most 15 µm, at most 10 µm or at most 5 µm.

Since the hydrophobic portion and the hydrophilic portion have different orientability each other with respect to the interface, it is possible to form macroemulsion particles and to provide a formulation having excellent feel of use. According to the related art, it is difficult for a molecular-level surfactant to provide stabilized macroemulsion particles having a particle diameter of several tens micrometers (µm) and the surfactant forms an interface film having a thickness of about several nanometers (nm). On the contrary, the amphiphilic anisotropic powder disclosed herein provides an interface film having a thickness increased to about several hundreds nanometers (nm) and forms a stabilized interface film by virtue of strong binding between powder particles, resulting in significant improvement in emulsion stability.

According to an embodiment, the cosmetic composition may be oil-in-water (O/W) type or water-in-oil (W/O) type or may have a multiple formulation of W/O/W or O/W/O. It is possible to provide various emulsion formulations merely by using the amphiphilic anisotropic powder disclosed herein. In addition, even in the case of an emulsion formulation having a high oil content, it has a powdery and matte fee of use without stickiness caused by the conventional surfactant.

The cosmetic composition may be an oil-in-water (O/W) type formulation with a weight ratio of the amphiphilic anisotropic powder:oil phase portion:aqueous phase portion of 0.1-15:5-60:10-80. In a variant, the cosmetic composition may be an oil-in-water (O/W) type formulation with a weight ratio of the amphiphilic anisotropic powder:oil phase portion:aqueous phase portion of 0.1-5:25-40:50-80. In another variant, the cosmetic composition may be a water-in-oil (W/O) type formulation with a weight ratio of the amphiphilic anisotropic powder:oil phase portion:aqueous phase portion of 1-15:50-80:10-30. The oil phase portion may include at least one selected from the group consisting of liquid oil and fat, solid oil and fat, wax, hydrocarbon oil, higher fatty acids, higher alcohols, synthetic ester oil and silicone oil.

According to still another embodiment, the amphiphilic anisotropic powder is added together with the aqueous phase portion to provide an emulsified cosmetic composition.

According to still another embodiment, the amphiphilic anisotropic powder may be used in an amount of 0.1-15 wt% based on the total weight of cosmetic composition. According to yet another embodiment, the amphiphilic anisotropic powder may be used in an amount of 0.5-5 wt% based on the total weight of cosmetic composition. Particularly, the amphiphilic anisotropic powder may be used in an amount of at least 0.1 wt%, at least 1 wt%, at least 2 wt%, at least 4 wt%, at least 6 wt%, at least 8 wt%, at least 10 wt% or at least 12 wt% and at most 15 wt%, at most 12 wt%, at most 10 wt%, at most 8 wt%, at most 6 wt%, at most 4 wt%, at most 2 wt% or at most 1 wt%, each based on the total weight of cosmetic composition. It is possible to control the size of emulsion particles from several micrometers (µm) to several tens or hundreds micrometers (µm) by adjusting the amount of amphiphilic anisotropic powder.

In another aspect, there is provided a method for preparing the cosmetic composition, including the steps of: (1) agitating a first monomer with a polymerization initiator to form a core of a first polymer spheroid; (2) agitating the resultant core of the first polymer spheroid with a first monomer, polymerization initiator and a functional group-containing compound to form a coated first polymer spheroid having a core-shell structure; (3) agitating the resultant first polymer spheroid having a core-shell structure with a second monomer and polymerization initiator to provide anisotropic powder in which a second polymer spheroid is formed; (4) introducing a hydrophilic functional group to the resultant anisotropic powder to provide amphiphilic anisotropic powder; and (5) carrying out emulsification by using the resultant amphiphilic anisotropic powder.

According to an embodiment, in steps (1)-(3), the agitation may be rotary agitation in a cylindrical reactor. Rotary agitation is preferred, since uniform mechanical mixing is required in addition to chemical modification so as to produce uniform particles. The rotary agitation may be carried out in a cylindrical rotary reactor but is not limited thereto.

Herein, the internal design of a reactor significantly affects formation of powder. In a cylindrical rotary reactor, the dimension and position of baffles, and the spacing of baffles from an impeller significantly affect the uniformity of the resultant particles. It is preferred that the spacing between internal baffles and impeller blades is minimized so as to homogenize the flow and intensity of convection, the reaction solution for powder is introduced to an extent smaller than the blade length, and the rotation speed of impeller is maintained at a high speed. The reactor may be rotated at a high speed of 200 rpm or more, have a ratio of diameter:height of 1-3:1-5, and more particularly, have a diameter of 10-30 cm and a height of 10-50 cm. The dimension may be varied with the reaction capacity. In addition, the cylindrical rotary reactor may be made of ceramic, glass or the like, and may be agitated at a temperature of 50-90°C.

In a cylindrical rotary reactor, a simple mixing process allows production of uniform particles, is a low-energy consuming process requiring low energy, and enables mass production through the maximization of reaction efficiency. According to the related art, a tumbling process in which a reactor itself rotates causes the whole reactor to be inclined at a predetermined angle and rotated at a high speed, and thus requires high energy and limits the dimension of a reactor. Due to the limitation in dimension of a reactor, the output is limited to a low degree of about several hundreds milligrams (mg) to several grams (g), and thus such a tumbling process is not suitable for mass production.

According to an embodiment, the first monomer and the second monomer may be the same or different, and particularly may be a vinyl monomer. In addition, the first monomer added in step (2) is the same as the first monomer used in step (1). The polymerization initiators used in each of the steps may be the same or different.

According to another embodiment, the vinyl monomer may be a vinyl aromatic monomer. For example, the vinyl monomer may be substituted or non-substituted styrene.

According to another embodiment, the polymerization initiator may be a radical polymerization initiator. Particularly, the polymerization initiator may be a peroxide initiator, azo initiator or a combination thereof. In addition, ammonium persulfate, sodium persulfate and potassium persulfate may be used.

According to another embodiment, in step (1), the first monomer may be mixed with the polymerization initiator at a weight ratio of 100-1000:1. According to still another embodiment, the first monomer may be mixed with the polymerization initiator at a weight ratio of 100-750:1, 100-500:1, or 100-250:1.

In still another aspect, in step (1), a stabilizer is further added in addition to the first monomer and polymerization initiator so that the first monomer, polymerization initiator and the stabilizer may be mixed at a weight ratio of 100-1000:1:0.001-5. The size and shape of powder is determined by controlling the size of the first polymer spheroid in the initial step (1) and the size of the first polymer spheroid may be controlled by the weight ratio of the first monomer, polymerization initiator and the stabilizer. In addition, it is possible to enhance the uniformity of anisotropic powder by mixing the compounds within the above-defined range.

According to still another embodiment, the stabilizer may be an ionic vinyl monomer, particularly sodium 4-vinyl benzene sulfonate. The stabilizer prevents the resultant particles from swelling, and imparts positive or negative charges to the surface of powder to prevent coalescence (binding) of particles electrostatically during the production thereof.

When the amphiphilic powder has a size of 200-250 nm, it is possible to obtain the powder from the first polymer spheroid in which the weight ratio of the first monomer:polymerization initiator:stabilizer is 110-130:1:1-5, particularly 115-125:1:2-4.

In addition, when the amphiphilic powder has a size of 400-450 nm, it is possible to obtain the powder from the first polymer spheroid in which the weight ratio of the first monomer:polymerization initiator:stabilizer is 225-240:1:1-3, particularly 230-235:1:1-3.

Further, when the amphiphilic powder has a size of 1100-2500 nm, it is possible to obtain the powder from the first polymer spheroid in which the weight ratio of the first monomer:polymerization initiator:stabilizer is 110-130:1:0 particularly 115-125:1:0.

Further, amphiphilic powder having an asymmetric snowman form may be obtained from the first polymer spheroid prepared at a weight ratio of the first monomer:polymerization initiator:stabilizer of 100-140:1:8-12, particularly 110-130:1:9-11.

Further, amphiphilic powder having an asymmetric reversed snowman form may be obtained from the first polymer spheroid prepared at a weight ratio of the first monomer:polymerization initiator:stabilizer of 100-140:1:1-5, particularly 110-130:1:2-4.

According to still another embodiment, in step (2), the functional group-containing compound may be siloxane-containing (meth)acrylate, particularly 3-(trimethoxysilyl)propyl acrylate, 3-(trimethoxysilyl)propyl methacrylate, vinyltriethoxysilane, vinyltrimethoxysilane or a combination thereof.

According to still another embodiment, in step (2), the first monomer, polymerization initiator and the functional group-containing compound may be mixed at a weight ratio of 80-98:0.2-1.0:1-20. According to still another embodiment, the first monomer, polymerization initiator and the functional group-containing compound may be mixed at a weight ratio of 160-200:1:1-6-40. The coating degree may be controlled by the weight ratio and the resultant amphiphilic anisotropic powder has a form determined by the coating degree. When the compounds are reacted at the above-defined weight ratio, the coating thickness is increased by about 10-30%, particularly about 20% based on the initial thickness. In this case, powder formation proceeds smoothly without non-powder formation caused by excessively thick coating or multi-directional powder formation caused by excessively thin coating. In addition, mixing within the above-defined weight ratio enhances the uniformity of anisotropic powder.

According to still another embodiment, in step (3), the second monomer and polymerization initiator may be mixed at a weight ratio of 150-250:1. According to still another embodiment, the second monomer and polymerization initiator may be mixed at a weight ratio of 160-250:1, or 170-250:1, or 180-250:1, or 190-250:1, or 200-250:1, or 210-250:1, or 220-250:1, or 230-250:1, or 240-250:1.

According to still another embodiment, in step (3), a stabilizer is added together with the second monomer and polymerization initiator so that the second monomer, polymerization initiator and the stabilizer may be mixed at a weight ratio of 150-250:1:0.001-5. Particular examples of stabilizer are the same as described above. In addition, mixing within the above-defined weight ratio enhances the uniformity of anisotropic powder.

According to still another embodiment, in step (3), the second monomer may be mixed in an amount of 40-300 parts by weight based on 100 parts by weight of the first polymer spheroid having a core-shell structure. Particularly, when the second monomer is used in an amount of 40-100 parts by weight based on 100 parts by weight of the first polymer spheroid having a core-shell structure, asymmetric snowman-type powder is obtained. When the second monomer is used in an amount of 100-150 parts by weight or 110-150 parts by weight, powder of a symmetric form is obtained. When the second monomer is used in an amount of 150-300 parts by weight or 160-300 parts by weight, asymmetric reversed snowman-type powder is obtained. In addition, mixing within the above-defined weight ratio enhances the uniformity of anisotropic powder.

According to still another embodiment, in step (4), the hydrophilic functional group may be introduced by using a silane coupling agent and a reaction modifier, but is not limited thereto.

According to still another embodiment, the silane coupling agent may be at least one selected from the group consisting of (3-aminopropyl) trimethoxysilane, N-[3-(trimethoxysilyl)propyl]ethylenediamine, N-[3-(trimethoxysilyl)propyl]ethylenediammonium chloride, (N-succinyl-3-aminopropyl)trimethoxysilane, 1-[3-(trimethoxysilyl)propyl]urea and 3-[(trimethoxysilyl)propyloxy]-1,2-propanediol, and particularly may be N-[3-(trimethoxysilyl)propyl]ethylenediamine.

According to still another embodiment, the silane coupling agent may be mixed in an amount of 35-65 parts by weight, for example 40-60 parts by weight, based on 100 parts by weight of the anisotropic powder obtained from step (3). Within the above-defined range, hydrophilization may be accomplished adequately.

According to still another embodiment, the reaction modifier may be ammonium hydroxide.

According to yet another embodiment, the reaction modifier may be mixed in an amount of 85-115 parts by weight, for example 90-110 parts by weight, based on 100 parts by weight of the anisotropic powder obtained from step (3). Within the above-defined range, hydrophilization may be accomplished adequately.

According to the related art, there have been attempts to increase the surface active property of spherical powder particles for use in Pickering by imparting amphiphilic surface activity thereto, and one example thereof includes Janus spherical particles. However, such particles are not practically applicable due to their geometrical limit and difficulty in mass production. On the contrary, the method for preparing amphiphilic powder disclosed herein uses no crosslinking agent and thus causes no agglomeration during the process, resulting in high yield and uniformity. In addition, the method disclosed herein uses a simple agitation process and is more amenable to mass production as compared to a tumbling process. Particularly, according to the method disclosed herein, it is possible to carry out mass production of nano-size particles having a size of 300 nm or less in a scale of several tens grams (g) to several tens kilograms (kg).

### [Mode for Invention]

The examples will now be described. It is apparent to those skilled in the art that the following examples are for illustrative purposes only and not intended to limit the scope of the present disclosure.

### Preparation Example 1. Preparation of Polystyrene (PS)-Based First Polymer Spheroid

In an aqueous phase, 40 g of styrene as a monomer, 1.0 g of sodium 4-vinylbenzene sulfonate as a stabilizer and 0.5 g of azobisisobutyronitrile (AIBN) as a polymerization are mixed and allowed to react at 75°C for 8 hours. The reaction is carried out by agitation in a cylindrical rotary reactor, which has a diameter of 11 cm and a height of 17 cm, is made of glass and is rotated at a speed of 200 rpm.

### Preparation Example 2. Preparation of Coated First Polymer Spheroid Having Core-Shell Structure

To 300 g of the polystyrene (PS)-based first polymer spheroid obtained as described above, 50 g of styrene as a monomer, 6 g of TMSPA (3-(trimethoxysilyl) propyl acrylate) and 0.2 g of azobisisobutyronitrile (AIBN) as a polymerization initiator are added and allowed to react at 75°C for 8 hours. The reaction is carried out by agitation in a cylindrical rotary reactor.

### Preparation Example 3. Preparation of Anisotropic Powder

To 24g of the aqueous dispersion of polystyrene-based core-shell (PS-CS) obtained as described above, 40 g of styrene as a monomer, 0.35 g of sodium 4-vinylbenzene sulfonate as a stabilizer, and 0.2 g of azobisisobutyronitrile (AIBN) as a polymerization initiator are added and heated to 75°C to carry out reaction for 8 hours. The reaction is carried out by agitation in a cylindrical rotary reactor.

### Preparation Example 4. Hydrophilization

To 600g of the aqueous dispersion of anisotropic powder obtained as described above, 30 g of N-[3-(trimethoxysilyl)propyl]ethylenediamine as a silane coupling agent and 60 g of ammonium hydroxide as a reaction modifier are added and reacted at 25°C for 24 hours to introduce a hydrophilic functional group. The reaction is carried out by agitation in a cylindrical rotary reactor.

### Examples 1-4

Emulsion cosmetic compositions are provided by using the amphiphilic anisotropic powder obtained as described above. Each of oil-in-water (O/W) type, water-in-oil (W/O) type, W/O/W and O/W/O multiple formulations is prepared. The particular compositions are shown in the following Tables 1, 2 and 3.

**[Table 1]**

| Wt% | Ex. 1(O/W) | Ex. 2(W/O) |
|---|---|---|
| Puresyn4 | 25 | 75 |
| CestosKD | 0.8 | 0.5 |
| DB | 0.3 | 0.25 |
| C981 | 0.2 | - |
| PE | 0.4 | 0.3 |
| TAU | 0.1 | - |
| Water | Up to 100 | Up to 100 |

**[Table 2]**

| Wt% | Ex. 3 (W/O/W) |
|---|---|
| Ex. 2(W/O) | 16 |
| DB | 0.5 |
| C981 | 1 |
| PE | 0.1 |
| TAU | 0.05 |
| Water | Up to 100 |

**[Table 3]**

| Wt% | Ex. 4 (O/W/O) |
|---|---|
| Puresyn4 | 85 |
| Ex. 1 (O/W) | 7 |
| DB | 0.5 |
| C981 | - |
| PE | 0.2 |
| TAU | - |
| Water | Up to 100 |

Puresyn4 : Hydrogenated poly(C6-14) olefin (oil)
CetosKD : Cetearyl alcohol (wax)
TAU : Tromethamine (acidity modifier)
PE : Phenoxyethanol (preservative)
C981 : Polyacrylate (thickener)
DB : Amphiphilic anisotropic powder

### Test Example 1

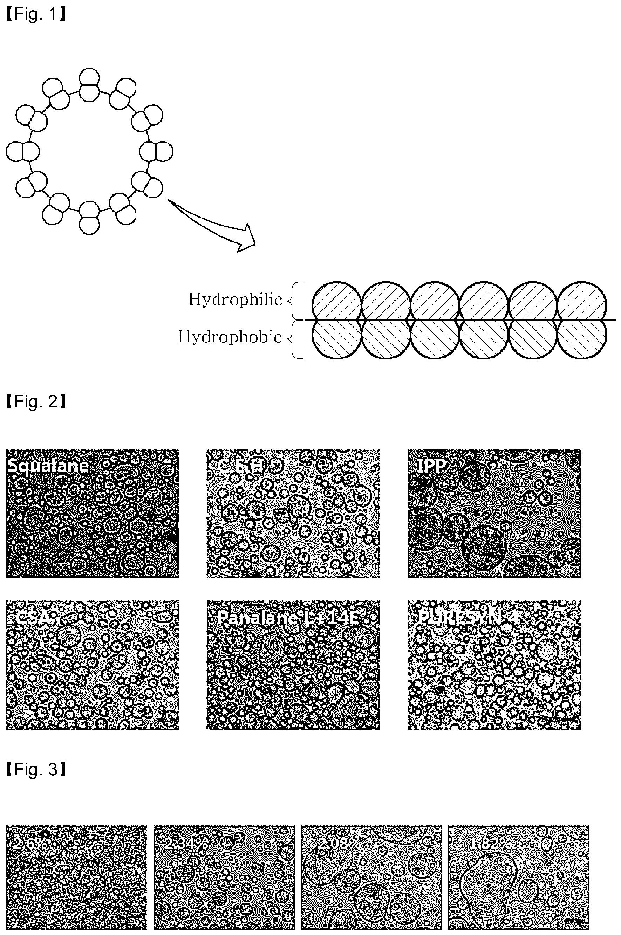

To an aqueous phase portion including the anisotropic powder dispersed in water, oil, wax, or the like are introduced as shown in Table 1. Then, emulsification is carried out to form O/W emulsion particles and the image of emulsion particles observed with an optical microscope is shown in Fig. 2. In addition, optical microscopic images of emulsions using each of 25% of squalane, cetyl octanoate (C.E.H), isopropyl palmitate (IPP), caprylic-capric triglyceride (CSA) and hydrogenated polyisobutene (Panalane L+14E) instead of 25% of hydrogenated poly(C6-14) olefin (Puresyn4) 25% are also shown.

As a result, it can be seen that even when using a small amount of anisotropic powder (0.3%), it is possible to form stabilized macroemulsion particles having a diameter of several tens micrometers (µm) with various compositions containing a different type of oil (ester oil, hydrocarbon oil, etc.).

### Test Example 2

In this Example, compositions are obtained in the same manner as Example 1, except that the amount of amphiphilic anisotropic powder is adjusted. Optical microscopic images of O/W emulsion particles formed with an amount of 2.6%, 2.34%, 2.08% or 1.82% are shown in Fig. 3.

After the test, it can be seen from Fig. 3 that the size of emulsion particles can be controlled from several micrometers (µm) to several tens or hundreds micrometers (µm) by adjusting the amount of amphiphilic anisotropic powder. As the amount of anisotropic powder increases, the surface area of interface film between an oil phase and an aqueous phase increases, resulting in a decrease in size of emulsion particles and an increase in number of emulsion particles.

### Test Example 3

In the compositions obtained from Example 1, variations in formulation are observed depending on oil/water ratio.

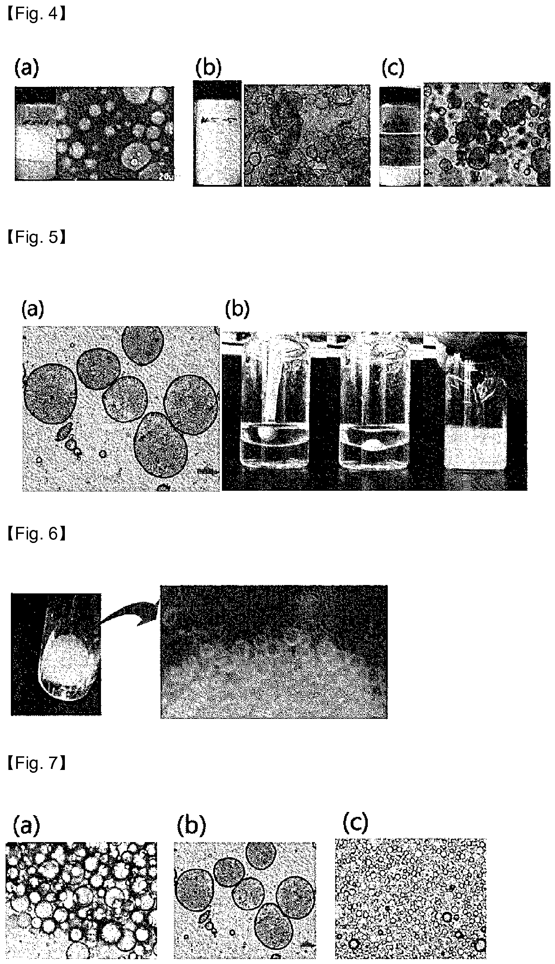

To confirm that various formulation characteristics are realized by varying the ratio of water/oil/anisotropic powder, formulations having a ratio of water/oil/anisotropic powder of (a) 75/20/5, (b) 38/60/2, or (c) 28/70/2 are observed and the results are shown in Fig. 4.

When a higher amount of aqueous phase is used, O/W emulsions are formed largely and the formed macroemulsion particles cause a creaming phenomenon in which the macroemulsion particles float toward the upper layer with time. On the contrary, when a higher amount of oil phase is used, W/O emulsions are formed largely and sedimentation occurs so that the particles settled down at the lower layer with time. When oil and water are used in a similar amount, uniform emulsion particles are formed as a whole. Therefore, it can be seen that controlling the amount of oil and water causes a variation in formulation of O/W or W/O.

### Test Example 4

A test for determining emulsification capability is carried out by using the emulsion obtained from Example 1. The condition of particles right after their emulsification using simple hand-shaking is observed with a microscope.

A composition is mixed in a glass vial and emulsified manually to obtain stabilized emulsion particles, and the results are shown in Fig. 5 and Fig. 6. In Fig. 5(a), it can be seen that the emulsion particles have a size of about 100 µm (concentration of anisotropic powder: 0.7%). In Fig. 5(b), the first vial shows the time point where aqueous dispersion of anisotropic powder is introduced to the interface between oil and water, the second vial shows anisotropic powder positioned at the oil/water interface, and the third vial shows formation of stabilized macroemulsion particles merely under light hand-shaking.

In addition, Fig. 6 shows formation of macroemulsion particles as observed by the naked eye. It can be seen that stabilized macroemulsion particles can be formed with ease merely under simple hand-shaking.

### Test Example 5

Fig. 7 shows the microscopic image of the emulsion particles in the emulsion obtained from Example 1. Each emulsion includes 25% of oil, Puresyn 4 (hydrogenated poly(C6-C14)olefin) and (a) 1% of the anisotropic powder, (b) 0.7% of the anisotropic powder, or (c) 1% of Tego Care 450 (polyglyceryl-3-methylglucose distearate). It can be seen from Fig. 7 that the emulsion particles have a significantly increased size (20-100 µm) as compared to the particle size (2-5 µm) obtained by using the conventional surfactant system.

### Test Example 6

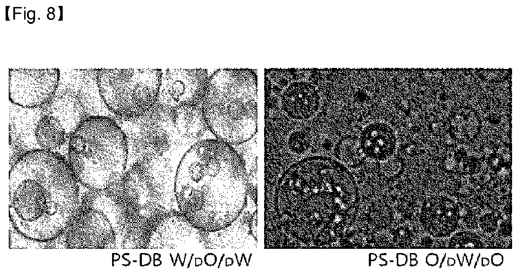

The emulsion obtained from Example 1 is subjected to repeated emulsification to obtain multiple formulations. Fig. 8 shows the optical microscopic images of Example 3 (W/O/W) and Example 4 (O/W/O).

While the exemplary embodiments have been shown and described, it will be understood by those skilled in the art that various changes in form and details may be made thereto without departing from the scope of this disclosure as defined by the appended claims. Therefore, it is intended that the scope of the present disclosure includes all embodiments falling within the spirit and scope of the appended claims.

## Claims

1. A cosmetic composition comprising amphiphilic anisotropic powder, wherein the powder comprises a first hydrophilic polymer spheroid and a second hydrophobic polymer spheroid,
the first polymer spheroid and the second polymer spheroid are bound with each other in such a manner that one polymer spheroid at least partially penetrates the other polymer spheroid, and
the first polymer spheroid has a core-shell structure, and the shell comprises a functional group.

2. The cosmetic composition according to claim 1, wherein the core of the first polymer spheroid and the second polymer spheroid comprise a vinyl polymer, and the shell of the first polymer spheroid comprises a copolymer of a vinyl monomer with a functional group.

3. The cosmetic composition according to claim 2, wherein the vinyl polymer is a vinyl aromatic polymer.

4. The cosmetic composition according to claim 1, wherein the functional group is a siloxane.

5. The cosmetic composition according to claim 1, wherein the shell of the first polymer spheroid further comprises a hydrophilic functional group introduced thereto.

6. The cosmetic composition according to claim 5, wherein the hydrophilic functional group is at least one selected from the group consisting of a carboxylic acid group, sulfone group, phosphate group, amino group, alkoxy group, ester group, acetate group, polyethylene glycol group and a hydroxyl group.

7. The cosmetic composition according to claim 1, wherein the amphiphilic anisotropic powder has a symmetric form, asymmetric snowman form or asymmetric reversed snowman form on the basis of the binding portion where the first polymer spheroid and the second polymer spheroid are bound with each other.

8. The cosmetic composition according to claim 1, wherein the amphiphilic anisotropic powder has a particle size of 100-2500 nm.

9. The cosmetic composition according to claim 1, wherein the amphiphilic anisotropic powder forms macroemulsion particles with a size of 2-200 µm.

10. The cosmetic composition according to claim 1, wherein the cosmetic composition is an emulsion composition having a formulation of an oil in water (O/W) type or water in oil (W/O) type, or a multiple formulation of W/O/W type or O/W/O.

11. The cosmetic composition according to claim 1, wherein the amphiphilic anisotropic powder is used in an amount of 0.1-15 wt% based on the total weight of cosmetic composition.

12. A method for preparing the cosmetic composition as defined in any one of claims 1 to 11, comprising the steps of:
(1) agitating a first monomer with a polymerization initiator to form a core of a first polymer spheroid;
(2) agitating the resultant core of the first polymer spheroid with a first monomer, polymerization initiator and a functional group-containing compound to form a coated first polymer spheroid having a core-shell structure;
(3) agitating the resultant first polymer spheroid having a core-shell structure with a second monomer and polymerization initiator to provide anisotropic powder in which a second polymer spheroid is formed;
(4) introducing a hydrophilic functional group to the resultant anisotropic powder to provide amphiphilic anisotropic powder; and
(5) carrying out emulsification by using the resultant amphiphilic anisotropic powder.

13. The method for preparing the cosmetic composition according to claim 12, wherein the agitation is carried out by rotary agitation in a cylindrical reactor, in steps (1)-(3).

14. The method for preparing the cosmetic composition according to claim 12, wherein the first monomer is mixed with the polymerization initiator at a weight ratio of 100-1000:1, in step (1).

15. The method for preparing the cosmetic composition according to claim 12, wherein the functional group-containing compound is siloxane-containing (meth)acrylate, in step (2).

16. The method for preparing the cosmetic composition according to claim 12, wherein the first monomer, polymerization initiator and the functional group-containing compound are mixed at a weight ratio of 80-98:0.2-1.0:1-20, in step (2).

17. The method for preparing the cosmetic composition according to claim 12, wherein the second monomer and polymerization initiator are mixed at a weight ratio of 150-250:1, in step (3).

18. The method for preparing the cosmetic composition according to claim 12, wherein the hydrophilic functional group is introduced by using a silane coupling agent, in step (4).
